(19) Europäisches Patentamt
European Patent Office
Office européen des brevets

(11) Numéro de publication: **0 066 684**
**B1**

(12) # FASCICULE DE BREVET EUROPEEN

(45) Date de publication du fascicule du brevet: **08.08.84**

(51) Int. Cl.³: **C 07 D 311/94**, C 11 B 9/00

(21) Numéro de dépôt: **82102757.0**

(22) Date de dépôt: **01.04.82**

(54) **Composés tricycliques oxygénés dérivés du norbornane et leur utilisation en tant qu'ingrédients parfumants.**

(30) Priorité: **21.05.81 CH 3315/81**

(43) Date de publication de la demande: **15.12.82 Bulletin 82/50**

(45) Mention de la délivrance du brevet: **08.08.84 Bulletin 84/32**

(84) Etats contractants désignés: **CH DE FR GB IT LI NL**

(56) Documents cités: **US - A - 4 159 258**

(73) Titulaire: **FIRMENICH SA, 1, route des Jeunes, CH-1211 Genève 8 (CH)**

(72) Inventeur: **Skorianetz, Werner, 545, route du Mandement, CH-1249 Dardagny (CH)**
Inventeur: **Ohloff, Günther, 13, chemin de la Chapelle, CH-1233 Bernex (CH)**

(74) Mandataire: **Salvadori, Giuseppe, Dr., c/o Firmenich S.A. Case Postale 239, CH-1211 Genève 8 (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

**Description**

L'invention a trait au domaine de la parfumerie et plus précisément à l'emploi d'un composé tricyclique oxygéné de formule (I)

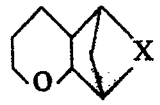

(I)

dans laquelle le symbole X représente un radical bivalent de formule

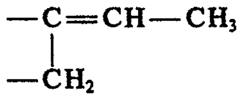

Ladite formule sert à désigner le 3-oxa-9-éthylidène-tricyclo[6.2.1.$0^{2,7}$] undécane ainsi que son isomère le 3-oxa-10-éthylidène-tricyclo[6.2.1.$0^{2,7}$] undécane, lesquels composés sont des entités chimiques nouvelles.

Parmi les composés présentant une structure chimique analogue à celles des composés de l'invention, figurent notamment les lactones tricycliques dérivées du norbornane définies par la formule générale que voici:

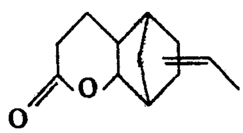

Ces composés font l'objet du brevet US N°. 4 159 258 et sont caractérisés par une odeur verte, fraîche, herbacée et légèrement grasse, parfois épicée.

Tout en présentant une note odorante qui, par certains de ses côtés, peut s'apparenter à celle des composés lactoniques décrits ci-dessus, les nouveaux composés de l'invention servent à développer des caractères olfactifs plus aromatiques, un peut âcres et dont la toralité verte et herbacée rappelle les fleurs du sureau. L'emploi des composés de formule (I) permet donc d'enrichir la palette mise à la disposition du parfumeur d'ingrédients synthétiques nouveaux présentant une note originale, jusque là inédite. Leur utilisation peut servir à la préparation de parfums de luxe qu'à celle de produits cosmétiques ou techniques tels les savons, les détergents ou les produits d'entretien par exemple.

Les proportions dans lesquelles les composés de l'invention peuvent être utilisés pour produire les effets odorants recherchés varient, comme souvent en pareils cas, dans une gamme de valeurs très étendue. Des concentrations de l'ordre de 0,1% en poids par rapport au poids du produit dans lequel ils sont incorporés, peuvent déjà produire des effets utiles. Ces concentrations cependant peuvent être comprises, dans la plupart des cas pratiques envisagés, entre environ 1 et 20%, leur détermination exacte peuvant être déduite par l'homme de l'art en fonction des effets recherchés, la nature des produits à parfumer et celle des autres ingrédients odoriférants présents dans une composition donnée. En effet, les composés de l'invention, tout en pouvant servir au parfumage de produits variés par addition directe, sont plus fréquemment employés sous forme de composition parfumante en mélange avec d'autres ingrédients, des solvants ou des supports.

Les composés de l'invention peuvent être préparés par réduction au moyen d'une hydrogénation catalytique des composés insaturés correspondants (II) obtenus par addition de l'acroléine au norbornène suivant le schéma réactionnel que voici:

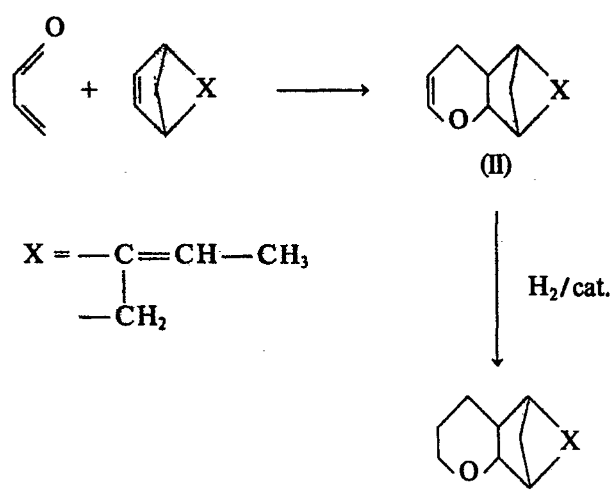


(II)

(I)

Les produits de départ (II), utilisés pour la préparation des composés de l'invention, peuvent être obtenus conformément au procédé décrit dans le brevet US N°. 4 159 258, par une réaction qui formellement constitue une cyclo-addition selon Diels-Alder à une température pouvant varier entre 150 et 250° C environ et à une pression comprise entre 15 et 150 atmosphères. Les composés (II) ainsi obtenus peuvent se présenter sous forme d'un mélange d'isomères, distincts l'un de l'autre par l'emplacement du radical éthylidényle. Pour toute utilisation pratique suivant la présente invention, il convient d'employer de tels mélanges afin d'obtenir, après réduction, des mélanges correspondants des composés (I), lesquels serviront directement au parfumage d'articles variés ou à la manufacture de compositions parfumantes.

La réduction desdits composés (II) peut s'effectuer suivant les techniques usuelles par hydrogénation catalytique en présence d'un catalyseur métallique usuel, par exemple le palladium sur charbon, la réduction étant interrompue après adsorption d'un équivalent d'hydrogène afin déviter la réduction du groupe éthylidène.

La formule (I) sert à désigner non seulement des isomères de position distincts vis-à-vis de l'emplacement du radical éthylidényle, mais également différentes formes stéréoisomériques de ceux-ci, formes qui peuvent être illustrées par les formules suivantes:

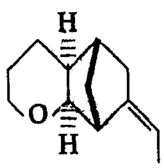
(a)

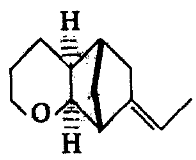
(b)

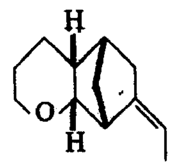
(c)

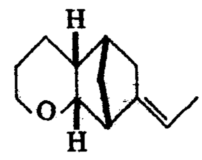
(d)

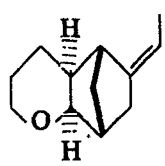
(e)

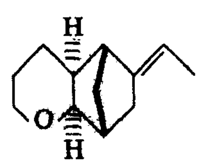
(f)

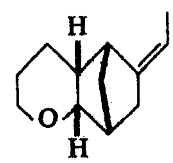
(g)

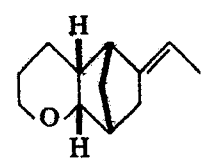
(h)

Par le procédé tel que décrit plus haut, on obtient un mélange isomérique contenant éminemment la paire isomérique de type exo- a/b et e/f dans des proportions respectives d'environ 45,5/13 et 32,5/9.

L'invention est illustrée par les exemples suivants dans lesquels les températures sont indiqués en degrés centigrades et les abréviations ont le sens usuel dans l'art.

## Exemple 1

### Préparation des composés de formule (I)

53 g (0,3 M) du produit obtenu par réaction d'éthylidène-norbornène avec l'acroléine [voir Exemple 1 du brevet US 4 159 258] ont été dissous dans 250 ml de méthanol et soumis à hydrogénation en présence de 3 g de palladium à 5% sur charbon. La réaction a été interrompue après addition d'un équivalent d'hydrogène (7,2 l), puis le mélange a été filtré et le filtrat clair distillé au moyen d'un appareil à boules (130°, température du bain/1,33 Pa). On a ainsi obtenu 52,3 g (rendement 98%) du produit désiré dont les caractères analytiques étaient les suivants:

| | |
|---|---|
| IR (film): | 3040, 2920, 1670, 1460, 1375, 1265, 1190, 1080, 1040, 989, 942, 906, 805, 642 $cm^{-1}$; |
| SM: | $M^+$ = 178(50); m/e: |
| | 163(17), 149(15), 135(9,4), 119(27), 106(16), 94(77), 93(100), 79(87), 67(18), 55(27), 41(42); |
| RMN (360 MHz): | signaux à 2,19; 2,60, 262 et 2,93 $\delta$ ppm |
| $^{13}C$: | 14,04; 79,29; 114,97; 141,48; 14,62; 78,12; 115,75; 140,51 |
| | 13,71; 79,81; 110,87; 145,12; 14,43; 79,90; 111,59; 144,3 $\delta$ ppm |

L'analyse spectroscopique nous a permis d'attribuer au mélange ainsi préparé la composition suivante:

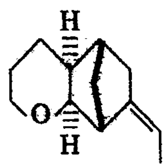 env. 46%; 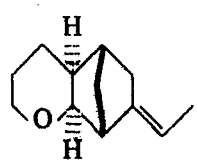 env. 13%

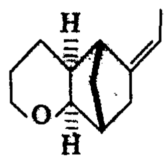 env. 32%; 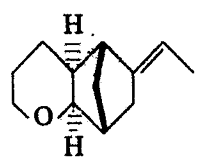 env. 9%

Exemple 2

Parfum pour shampooing

Une composition parfumante de base destinée au parfumage de shampooings a été préparé en mélangeant les ingrédients suivants (parties en poids):

| | |
|---|---|
| Triméthyl-hexyle acétate | 200 |
| Géranylacétone | 100 |
| Salicylate de benzyle | 80 |
| Aldéhyde hexylcinnamique | 80 |
| Isobutyrate de 1,3-diméthyl-but-3-ène-1-yle | 80 |
| p-tert-Butyl-$\alpha$-méthyl hydrocinnamaldéhyde | 60 |
| Alcool phényléthylique | 60 |
| $\alpha$-Isométhylionone | 40 |
| Essence d'Ylang-Ylang | 40 |
| 4-Isopropyl-cyclohexylméthanol[1]) | 40 |
| Alcool hydratropique | 40 |
| Aldéhyde anisique à 10%* | 30 |
| Alcool anisique | 20 |
| Phénoxyacétate d'allyle | 10 |
| Géraniate de méthyle | 10 |
| Acétate de trichloro-méthyl-phényl-carbinyle cristallisé | 10 |
| $\alpha$-Damascone à 10%* | 10 |
| Exaltex® à 10%*[2] | 10 |
| Isobutyrate de phénoxyéthyle | 10 |
| Triméthyl-cyclohexène-carboxyaldéhyde à 10%* | 10 |
| Total | 940 |

* dans le phtalate diéthylique
1) brevet US 3 993 604; Mayol® (origine Firmenich SA)
2) Cyclopentadécanolide

La composition de base ainsi obtenue présente un caractère fleuri-frais. Par addition à cette composition de 60 g du mélange isomérique de 3-oxa-9-éthylidène-tricyclo[6.2.1.$0^{2,7}$] undécane et 3-oxa-10-éthylidène-tricyclo[6.2.1.$0^{2,7}$] undécane obtenu suivant l'Exemple 1, on a obtenu une nouvelle composition présentant une note caractéristique et montante de type fleurs de sureau.

**Revendications**

1. Composé de formule (I)

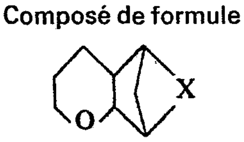

(I)

dans laquelle le symbole X représente un radical bivalent de formule

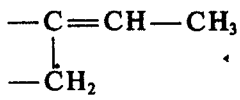

2.3-Oxa-9-éthylidène-tricyclo[6.2.1.$0^{2,7}$]undécane.

3. 3-Oxa-10-éthylidène-tricyclo[6.2.1.$0^{2,7}$]undécane.

4. Composé suivant la revendication 3, sous forme de l'un quelconque des isomères de formule

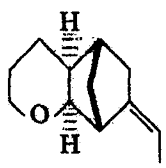

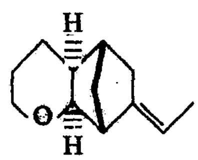

(a) (b)

5.Composé suivant la revendication 2, sous forme de l'un quelconque des isomères formule

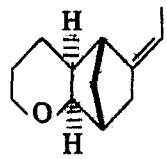

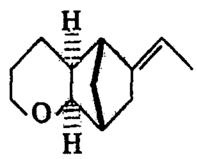

6. Utilisation d'un composé suivant l'une des revendications 1 à 5 en tant qu'agent parfumant pour la préparation de parfums et produits parfumés.

7. Composition parfumante caractérisée en ce qu'elle contient en tant qu'ingrédient actif un composé suivant l'une des revendications 1 à 5.

8. Procédé pour la préparation d'un composé suivant l'une des revendications 1 à 5, caractérisé en ce qu'on réduit au moyen d'une hydrogénation catalytique un composé de formule

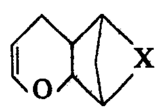

(II)

dans laquelle le symbole X représente un radical bivalent de formule

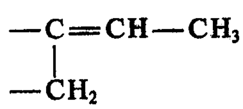

**Patentansprüche**

1. Verbindung der Formel (I)

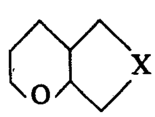

(I)

worin das Symbol X einen bivalenten Rest der Formel

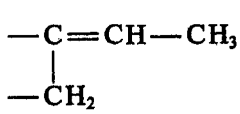

bedeutet.

2. 3-Oxa-9-ethyliden-tricyclo[6.2.1.$0^{2,7}$]undecan.

3. 3-Oxa-10-ethyliden-tricyclo[6.2.1.$0^{2,7}$]undecan.

4. Verbindung gemäß Anspruch 3, unter der Form eines der Isomeren der Formeln

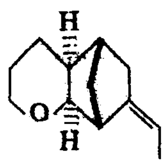 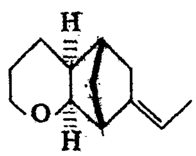

(a) (b)

5. Verbindung gemäß Anspruch 2, unter der Form eines der Isomeren der Formeln

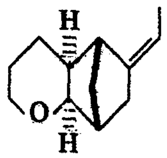 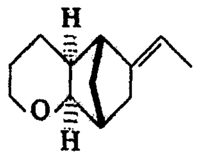

(e) (f)

6. Verwendung von einer Verbindung gemäß einem der Ansprüche 1 bis 5, als geruchstragender Bestandteil zur Herstellung von Parfümen und parfümierten Produkten.

7. Parfüm Komposition, dadurch gekennzeichnet, daß sie eine der Verbindungen gemäß einem der Ansprüche 1 bis 5 als aktiven Bestandteil enthält.

8. Verfahren zur Herstellung einer Verbindung gemäß einem der Ansprüche 1 bis 5, dadurch gekennzeichnet, daß man eine Verbindung der Formel

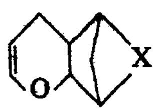

(II)

worin das Symbol X einen bivalenten Rest der Formel

$-C{=}CH-CH_3$
$-CH_2$

bedeutet, katalytisch hydriert.

**Claims**

1. Compound of formula

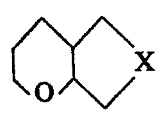

(I)

wherein symbol X represents a divalent radical of formula

$-C{=}CH-CH_3$
$-CH_2$

2. 3-Oxa-9-ethylidene-tricyclo[6.2.1.$0^{2,7}$]undecane.
3. 3-Oxa-10-ethylidene.tricyclo[6.2.1.$0^{2,7}$]undecane.
4. Compound according to claims 3 in the form of anyone of the isomers of formulae

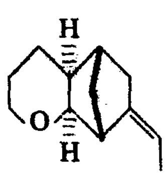 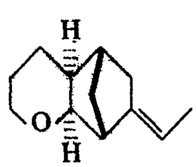

(a) (b)

5. Compound according to claim 2 in the form of anyone of the isomers of formulae

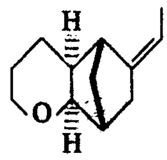

(e)

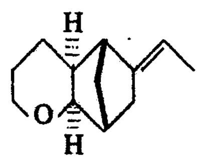

(f)

6. Utilization of a compound according to one of the claims 1 to 5 as perfuming agent for the preparation of perfumes and perfumed products.

7. Perfume composition characterized in that it cortains as active ingredient a compound according to one of the claims 1 to 5.

8. Process for the preparation of a compound according to one of the claims 1 to 5, characterized in that a compound of formula

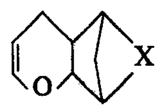

(II)

wherein symbol X represents a divalent radical of formula

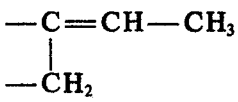

is reduced by catalytic hydrogenation.